# FASCICULE DE BREVET EUROPEEN

(11) **EP 0 777 972 B1**
(45) Date de publication et mention de la délivrance du brevet: **14.05.2003**
(21) Numéro de dépôt: 95203391.8
(22) Date de dépôt: 07.12.1995
(51) Int. Cl.: A23F 3/16, C12N 11/00

(54) **Préparation d'un extrait de thé**
Herstellung von Tee-Extrakt
Producing a tea extract

(43) Date de publication de la demande: 11.06.1997
(73) Titulaire: SOCIETE DES PRODUITS NESTLE S.A., 1800 Vevey (CH)
(72) Inventeur: Nicolas, Pierre, CH-1806 Saint-Legier (CH); Raetz, Eric, CH-1012 Lausanne (CH); Reymond, Sylviane, CH-1066 Epalinges (CH); Sauvageat, Jean-Luc, CH-1018 Lausanne (CH)

(56) Documents cités:
- EP-A- 0 391 468
- GB-A- 1 249 932
- GB-A- 1 380 135
- US-A- 3 959 497
- US-A- 4 217 338
- BIOTECHNOLOGY AND BIOENGINEERING, vol. 16, no. 8, 1974 pages 1095-1102, XP 000569188 H. WEETALL ET AL. 'Immobilized Tannase'
- BIOTECHNOLOGY AND BIOENGINEERING, vol. 26, 1984 pages 1223-1226, XP 000568881 M. MAREK ET AL. 'Invertase immobilisation via its carbohydrate moiety'

## Description

La présente invention concerne toute tannase immobilisée covalemment par sa partie glycosidique sur un support insoluble, ainsi qu'un nouveau procédé de préparation d'un extrait de thé permettant de solubiliser la crème de thé.

### Etat de la technique

Le thé noir, utilisé pour la préparation de boissons chaudes et froides, est préparé traditionnellement en soumettant des feuilles vertes fraîches de thé à différents traitements, dont une étape de fermentation par des enzymes oxydatives des feuilles qui est indispensable à l'obtention des composés conférant les qualités organoleptique et de couleur caractéristiques du thé noir. Lorsque l'on extrait à l'eau chaude du thé noir, un précipité apparaît par refroidissement à des températures inférieures à 55°C, voire même 60°C, dû à la formation de complexes partiellement insolubles d'esters de polyphénols et de caféine, qui est désigné communément par l'expression "crème de thé" ou "crème".

La préparation d'une boisson froide à base de thé noir nécéssite ainsi à l'élimination de la crème de thé. EP198209 (Société des produits Nestlé S.A.) propose ainsi de soumettre des feuilles de thé noir à une extraction à l'eau à 60-130°C, ce qui donne un premier extrait qui est séparé des feuilles de thé, concentré à une teneur en matières solides de 5-12,5% puis refroidi à 5-15°C pour former une crème insoluble qui est séparée du premier extrait concentré, puis est soumise à une extraction à l'eau à 40-70°C, ce qui donne un second extrait qui est séparé de la crème insoluble restante, les premier et second extraits étant ensuite mélangés. L'inconvénient de ce procédé est que des matières solides sont éliminées. De plus, l'élimination de la crème engendre une perte d'arôme non-négligeable.

Pour remédier à ces problèmes, il a été envisagé d'utiliser une tannin acyl hydrolase, communément appellée tannase, pour hydrolyser les esters de polyphénols, notamment les gallates de polyphénols qui se complexent avec la caféine pour former la crème de thé. Bien que les tannases permettent effectivement de réduire substantiellement la formation de crème de thé, leur utilisation se heurte au coût élevé des enzymes ainsi qu'à leur instabilité à des températures supérieures à 40°C (Thomas R.L. et al., Journal of Food Science, 50, 1126-1129, 1985).

Afin de pallier le coût et l'instabilité de l'enzyme, GB1380135 (Unilever N.V.) propose de traiter à chaud un extrait aqueux de thé par des tannases immobilisées covalemment par leur partie peptidique sur un support insoluble. Les températures utilisées sont généralement de l'ordre de 50°C, de façon à pouvoir traiter la crème avant précipitation. Malheureusement, l'enzyme immobilisée perd substantiellement son activité après quelques passages de son substrat, ce qui est économiquement et industriellement peu rentable. D'autres désavantages sont mentionnés dans EP0391468 (Unilever N.V.), comme une solubilisation insuffisante de la crème. Un relarguage important des enzymes du support est aussi fréquemment constaté.

De même, US4051264 (Lipton Inc.) propose de traiter directement des feuilles de thé vertes émincées avec des tannases libres ou immobilisées covalemment par leur partie peptidique, en anaérobiose, à une température et pendant un temps suffisants pour améliorer l'extractibilité des feuilles à froid, de fermenter les feuilles pour obtenir des feuilles de thé noir, et de les chauffer jusqu'à ce que leur teneur en humidité soit inférieure à 5%. On peut aussi noter que la température de réaction des enzymes immobilisées ne peut être supérieure à 45°C sans occasionner une perte importante d'activité enzymatique (voir le tableau 4 du brevet susmentionné).

### Résumé de l'invention

La présente invention a but de pallier les inconvénients de l'art antérieur. A cet effet, l'invention concerne toute tannase immobilisée covalemment par sa partie glycosidique sur un support insoluble.

L'invention concerne aussi un procédé de préparation d'un extrait de thé, dans lequel on prépare un extrait aqueux de feuilles de thé, et on traite l'extrait à une température de 20-65°C par des tannases dont la partie glycosidique est immobilisée covalemment sur un support insoluble.

Les tannases immobilisées selon l'invention présentent l'avantage d'être particulièrement stables dans le temps à des températures supérieures à 50°C ou 55°C, voire même 60°C. On a pu ainsi montrer que la tannase selon l'invention présente une température critique à partir de laquelle elle devient instable, qui est de 15°C à 25°C supérieure à celle de l'enzyme libre, ce qui est particulièrement exceptionnel dans le domaine des enzymes immobilisées.

Un autre avantage réside dans le fait que l'on n'observe pas de relarguage de la tannase du support et que l'enzyme est encore active après au moins 400 passages de son substrat. Cette stabilité dans le temps est très surprenante et n'est pas du tout prévisible, en particulier lorsqu'on la compare à celle de la tannase immobilisée de GB1380135 ou US4051264.

Un avantage du présent procédé est que l'on peut travailler à une température qui est défavorable à un développement bactérien, améliorant ainsi les conditions d'hygiène microbiologique du procédé.

Un autre avantage du présent procédé est qu'à des températures de 55°C à 70°C, la crème de thé est particulièrement soluble, ce qui permet aux tannases immobilisées de mieux hydrolyser les complexes polyphénoliques. On évite ainsi le difficile, sinon impossible, transfert de matière solide, ce qui améliore nettement la vitesse et le rendement final de solubilisation de la crème.

Enfin, la présente invention présente encore l'avantage que l'on peut hydrolyser un extrait de thé dans un réacteur de type cuve agitée et dans un réacteur comprenant un lit fixe ou un lit fluidisé de tannases immobilisées.

Dans la suite de la description, on entend par "réacteur de type cuve agitée" un système agité mécaniquement comprenant en suspension des tannases immobilisées. On entend également par "un lit fixe de tannases immobilisées" des tannases immobilisées sur ou dans un support qui est compacté dans un réacteur destiné au traitement en continu d'un extrait liquide de thé. De même, on entend par "un lit fluidisé de tannases immobilisées" des tannases immobilisées sur un support, qui est placé mais non compacté dans un réacteur destiné au traitement en continu d'un extrait liquide de thé. Le flux d'extrait de thé, circulant de bas en haut, met alors les particules de support en suspension.

Par thé noir et thé Oolong, on entend respectivement le produit de l'oxydation enzymatique complète et partielle de feuilles de thé vert obtenue à partir de l'arbre à thé *Camellia* notamment C. *sinensis* et C. *assaimica*. Les membres du genre *Phyllanthus*, *Catechu*, *Gambir* ou *Uncaria* peuvent être aussi utilisés.

Enfin, on définit l'unité de tannase comme étant la quantité de tannase qui hydrolyse 1µmole de gallate de méthyle en 1 min à pH5 et à 30°C.

### Description détaillée de l'invention

Pour mettre en oeuvre le présent procédé, on extrait à l'eau des feuilles de thé provenant de toute espèce de l'arbre à thé, et plus particulièrement des feuilles de thé noir ou Oolong. L'extrait aqueux de thé peut ainsi être préparé par toute méthode connue de l'homme du métier pour extraire des feuilles de thé. Par exemple, on peut utiliser 2 à 25 parties en poids d'eau, de préférence 4 à 15 parties en poids, et en particulier 5 à 12 parties en poids par partie en poids sec de feuilles de thé. La durée de l'extraction peut être conventionnelle, par exemple jusqu'à 30 min, de préférence de 2 à 15 min et particulièrement de 5 à 12,5 min. La température de l'eau peut être conventionnelle, par exemple jusqu'à 130°C, de préférence de 60°C à 125°C, et en particulier de 75°C à 120°C, voire même de 85°C à 110°C.

L'extraction des feuilles peut être conduite dans une cuve à agitation comprenant l'eau et les feuilles. L'extraction peut aussi être réalisée en continu, par percolation ou dans un système à contre-courant dans lequel l'eau passe dans le sens inverse de la pesanteur dans des cellules comprenant les feuilles de thé. L'enseignement relatif à la préparation d'un extrait de thé décrit dans EP198209, EP481262, EP654221 et EP95202228.3 est incorporé par référence dans la description de la présente invention.

On peut séparer ensuite les feuilles de thé, et traiter l'extrait aqueux comprenant 1% à 11% en poids de solides par tous les procédés connus de l'homme du métier, comme par exemple procéder à une concentration jusqu'à obtenir 5 à 15% en poids d'extraits de thé. On peut aussi concentrer la partie crème de l'extrait, en refroidissant l'extrait à 4°C-20°C pour faire apparaître la crème, puis en séparant la crème par filtration ou centrifugation, par exemple. On peut aussi procéder à une ressolubilisation à l'eau chaude de la crème séparée, de préférence à 60°C-80°C. On peut aussi procéder à une extraction des arômes qui seront réincorporés dans l'extrait en fin de procédé, par exemple.

Finalement l'extrait aqueux comprenant la crème que l'on veut solubiliser par des tannases peut présenter une teneur en matière sèche de 1% à 30%, de préférence 1 à 20%.

On traite ensuite l'extrait par des tannases dont la partie glycosidique est immobilisée covalemment sur un support insoluble. La tannase utilisée est une glycoprotéine connue pour hydrolyser les esters d'acide gallique des composants polyphénoliques du thé. La tannase peut provenir de certains champignons du genre *Aspergillus* comme par exemple *Aspergillus niger*, *Aspergillus flavus* ou *Aspergillus oryzae*, du genre *Penicillium* comme par exemple *Penicillium chrysogenum*, ou encore des levures du genre *Candida*. Deux souches de microorganismes sont connues pour produire des quantités substantielles de tannase, comme *Aspergillus oryzae* ATCC No. 9362 et *Aspergillus niger* ATCC No. 16888. Des tannases peuvent être également obtenues du commerce sous une forme purifiée en poudre, par exemple de la société Enzyme Development Corporation (Tannase S®, N.Y., USA), ou Kikkoman (Tannase Kikkoman 50000U/g®, Japon).

De préférence, le support choisi présente des pores ayant un diamètre moyen de l'ordre de 20-2000 nm, notamment 50-1000 nm. Il peut être un support inorganique pouvant être choisi dans le groupe formé par les particules de silice, de verre, d'oxydes métalliques comme l'alumine, ou de minéraux naturels comme la bentonite, par exemple. Il peut être aussi un support organique pouvant être choisi dans le groupe formé par les polysaccharides comme la cellulose, le dextran ou la chitine, les protéines comme la soie, ou les polymères synthétiques du type polystyrène, polyacrylate, polyméthacrylate comme l'Eupergit® (Röhm, Allemagne), polyvinyle, et polyamide comme le nylon, par exemple.

Le support peut être activé en y introduisant des groupements capables de se lier à la partie glycosidique oxydée de la tannase, par exemple des groupements aminés libres ou des groupements hydrazides. De nombreuses méthodes d'activation de supports sont connues de l'homme de l'art, qui saura ainsi choisir en fonction du support les groupements actifs qui sont adaptés pour se lier avec la partie glycosidique de la tannase. A titre d'exemple, la méthode d'immobilisation décrite par Marek et al. pourra être suivie (Biotechnology and Bioengineering, 26, 1223-1226, 1984).

On peut modifier au préalable la tannase, par exemple en l'oxydant d'une manière conventionnelle. On peut ainsi l'oxyder avec une solution de 0,01-1M de periodate à pH 4-6 pendant 1 à 120 min à une température de 0°C à 25°C. La réaction d'oxydation peut être arrêtée par addition d'une solution réductrice, comme par exemple une solution à 1-3M d'éthylène glycol. De préférence la tannase oxydée est ensuite nettoyée des solutions d'oxydation ou d'arrêt, par exemple par dialyse dans un tampon adapté, notamment un tampon 0,01-1M de phosphate de sodium pH 5-7.

On peut alors immobiliser la tannase oxydée sur un support capable de réagir avec les groupements aldéhydes ainsi créés. Pour ce faire, on peut modifier de l'Eupergit-C® par une solution de 0,1-1M de dihydrazide adipique à pH 4-9, par une solution de 0,1-1M d'éthylène diamine pH 4-9, ou par une solution de 0,1-1M d'hexaméthylène diamine pH 4-9, par exemple. Pour cela, on peut mélanger 1 g équivalent en poids sec de support modifié à au moins 10 mg de tannase oxydée, de préférence 20-300mg pendant 1 à 30 h, à 4-30°C, par exemple.

Dans le cas du couplage par des diamines, on peut également consolider la liaison imine par réduction avec du borohydrure ou du cyanoborohydrure de sodium.

En définitive, il est préférable d'inactiver les groupes fonctionnels restants par des techniques connues de l'homme du métier. Toutefois cette étape d'inactivation n'est pas essentielle pour obtenir une tannase immobilisée active.

La tannase peut être immobilisée sur le support à raison de au moins 10 mg de tannase par g de support, de préférence 20-100 mg. L'activité de la tannase immobilisée peut être d'au moins 40 unités de tannase par g de support, par exemple 40-200 unités.

Lorsqu'on hydrolyse un extrait de thé avecla tannase immobilisée selon l'invention, on peut observer en début d'utilisation de l'enzyme, une adsorption de composants du thé sur le support. Ces composants peuvent malheureusement être impliqués dans la couleur et le goût du thé. Il peut donc être préférable d'utiliser une tannase immobilisée selon l'invention ayant été préincubée au moins une fois dans un extrait de thé.

Dans un premier mode préféré de la présente invention, on hydrolyse l'extrait de thé dans un réacteur de type cuve agitée, avec une tannase immobilisée selon l'invention. Le système peut être automatisé et fonctionner en mode semi-continu avec les trois phases suivantes. Premièrement, on remplit la cuve avec l'extrait de thé à traiter, puis on hydrolyse sous agitation mécanique, enfin on vidange la cuve après une durée déterminée. La rétention de l'enzyme dans le réacteur peut se faire au moyen d'un filtre placé dans la partie inférieure de la cuve. Ce filtre peut retenir toutes les particules de diamètre supérieur à 40 µm, par exemple. Pour traiter l'extrait de thé, on peut mélanger dans la cuve 1 partie en poids d'extrait à moins de 1 partie en poids de support, par exemple 0,0001-0,1 partie de support, puis incuber le mélange sous agitation à 20-65°C pendant 10-200 min, de préférence 50-65°C, voire 55-65°C, pendant 10-100 min, par exemple.

Selon ce mode d'hydrolyse, on peut observer que les tannases immobilisées selon l'invention présentent une remarquable stabilité, car elles conservent 100% de leur activité enzymatique après de nombreuses réutilisations jusqu'à 60°C, notamment pendant 5 à 420 cycles de 30 min à une température de 30°C à 60°C, les grands nombres de cycles correspondant aux petites températures et inversement. Enfin à 65°C l'enzyme perd lentement son activité au cours du temps, ce qui marque de préférence sa limite supérieure d'utilisation.

Dans un deuxième mode de réalisation préféré de la présente l'invention, on hydrolyse en continu l'extrait de thé liquide dans un réacteur comprenant un lit fixe de tannases immobilisées selon l'invention. De préférence, on fait passer au moins 1 partie en poids d'extrait par partie en poids de lit, par exemple 10-10000 parties d'extrait, à une température de 20-65°C, de préférence 50-65°C, voire 55-65°C.

Dans un troisième mode de réalisation préféré de la présente invention, on hydrolyse en continu l'extrait de thé liquide dans un réacteur comprenant un lit fluidisé de tannases immobilisées selon l'invention. De préférence, on fait passer au moins 1 partie en poids d'extrait par partie en poids de lit, par exemple 10-10000 parties d'extrait, à une température de 20-65°C, de préférence 50-65°C, voire 55-65°C.

L'extrait ainsi traité peut encore présenter des insolubles que les tannases n'ont pu solubiliser même après hydrolyse totale par les tannases immobilisées selon l'invention. Il peut donc s'avérer nécessaire de les séparer par filtration ou centrifugation après avoir refroidi l'extrait à 4-20°C, par exemple. Cette opération appelée classiquement "polishing" a déjà été décrite dans GB1380135 et EP391468.

L'extrait traité peut ensuite être utilisé traditionnellement dans la préparation d'une poudre de thé instantanée. Si l'extrait provient directement d'un extrait brut de feuilles de thé, on peut le concentrer et le sécher, par exemple par lyophilisation ou par atomisation, par exemple. Si l'extrait provient de la crème de thé préalablement séparée d'un extrait brut de feuilles de thé, on peut le mélanger à l'extrait brut, puis le concentrer et le sécher.

La poudre de thé reconstituée avec de l'eau comprend de préférence 0,25-0,3% en poids de solides de thé, et présente un pH 4,5-5,5. Elle présente une turbidité, mesurée à 10°C en "Unité de Turbimétrie Néphélométrique" (UTN), par exemple avec un turbimètre Hatch Ratio Turbimeter® (Hatch Company, Colorado, USA), qui est inférieure à 50 UTN, de préférence inférieure à 35 UTN, par exemple 5 à 15 UTN. La poudre de thé obtenue par le procédé selon la présente invention est un produit comprenant uniquement des extraits de thé, qui est instantanément soluble dans l'eau froide donnant une boisson dont la couleur et le goût sont particulièrement stables dans le temps et appréciés des dégustateurs.

Les exemples ci-après sont donnés à titre d'illustration du procédé et de la tannase immobilisée selon la présente invention. Dans ces exemples, on a utilisé la tannase commercialisée sous la marque Kikkoman 50000U/g®, et dans certains de ces exemples on a utilisé comme substrat modèle une solution de gallate de méthyle qui permet d'évaluer de façon commode et précise l'excellente stabilité de la préparation de tannase immobilisée selon l'invention. Ces exemples sont précédés d'une méthode de détermination de l'activité enzymatique de la tannase, d'une méthode de mesure d'acide gallique dans le thé, et d'une présentation des dessins.

### Activité enzymatique de la tannase

L'activité de la tannase est déterminée par la mesure en chromatographie liquide haute performance (HPLC) de l'acide gallique libéré par l'hydrolyse du gallate de méthyle. Pour cela, on mélange 0,5 ml de solution d'enzyme à 1,5 ml d'une solution de 20 mM de gallate de méthyle dans 50 mM d'un tampon acétate pH5. En pratique, les échantillons d'enzymes sont dilués avant l'incubation de manière à ce que la concentration en acide gallique ne dépasse pas 1mM en fin de réaction.

On incube le mélange à 30°C pendant 15 min, on arrête la réaction par 0,2 ml d'HCl 2M, on injecte 10 µl dans une colonne en phase inverse KS 250/6/4 Nucleosil® 100-5 C18 (Macherey-Nagel GmbH, Düren, DE) ayant comme phase mobile 2% d'acide acétique: acétonitrile (78:22 v/v), et on élue dans des conditions isocratiques, à une vitesse de 1 ml/min, avec une détection spectrophotométrique à 278 nm.

### Mesure de l'acide gallique

L'acide gallique produit dans le thé par la tannase est déterminé par HPLC avec la même colonne que celle décrite ci-dessus, en utilisant le gradient suivant, à une vitesse de 1 ml/min: 0-5 min: 88% A et 12% B; 5-16 min: 75%A et 25% B; 16-25 min 88% A et 12% B (A= acide acétique 2%; B= acétonitrile).

### Dessins

Fig. 1: libération d'acide gallique en fonction du nombre de cycles d'hydrolyse dans un réacteur du type "cuve agitée" à 30°C.

Fig. 2: libération d'acide gallique en fonction du nombre de cycles d'hydrolyse dans un réacteur du type "cuve agitée" à des températures variant de 30°C à 65°C.

### Exemples comparatifs

Un moyen classique d'immobiliser les enzymes sur l'Eupergit® est de faire réagir les résidus amine de la partie protéique de l'enzyme avec les groupes oxirane du support. La réaction est initiée par des concentrations en phosphate de 1M dans des conditions similaires à celles présentées dans l'exemple 3 de EP676145.

Nous avons pu remarquer qu'il n'est pas possible d'immobiliser la tannase Kikkoman 50000U/g® sur différents support d'Eupergit® dans les conditions présentées dans le tableau 1 ci-après, et cela même lorsque l'on dialyse la tannase pour la purifier ou lorsqu'on utilise de l'Eupergit-C® à plus larges pores ou non poreux pour permettre un accès plus facile de la tannase aux groupes oxiranes du support.

La tannase n'est donc pas une enzyme que l'on peut facilement immobiliser covalemment par sa partie peptidique. Ceci peut expliquer en partie le fait que les tannases immobilisées décrites dans l'art antérieur présentent des désavantages qui les rendent industriellement et économiquement peu attractives (voir EP391468).

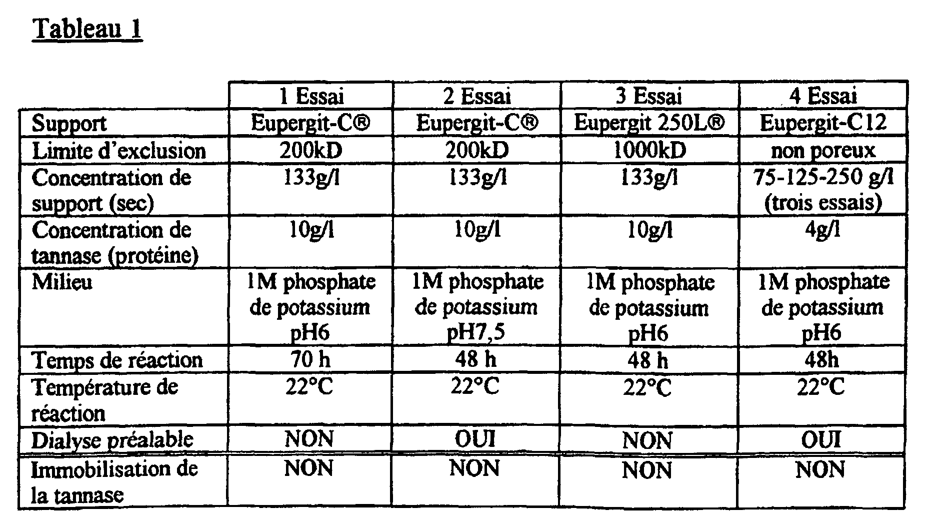

### Exemple 1

On oxyde au periodate la tannase Kikkoman 50000U/g® dans des conditions contrôlées. Pour cela, on mélange à 0,5 ml d'un tampon 0,1 M acétate de sodium pH 5,5 comprenant 0,1M de periodate de sodium, 4,5 ml d'un tampon 0,1 M acétate de sodium pH5,5 comprenant 22,22 g/l d'une poudre de tannase. On laisse le mélange s'oxyder à 0°C pendant 60 min à l'obscurité, on arrête la réaction par l'ajout de 0,5 ml d'une solution 2M d'éthylène glycol, puis on dialyse le mélange dans un tampon 0,1 M phosphate de sodium pH6 à 4°C.

D'un autre coté, on mélange à 1g d'Eupergit-C® sec, 20 ml d'un tampon 0,2 M phosphate de sodium comprenant 0,2 M de dihydrazide adipique pH 8. On incube le mélange à température ambiante sous une légère agitation pendant 16 h, puis on lave abondamment le support avec de l'eau distillée puis avec un tampon 0,1 M phosphate de sodium pH 6.

On mélange ensuite 1g en équivalent de poids sec d'Eupergit-C® modifiée humide à 5,4 ml de solution de tannase oxydée, on incube à 4°C sous une légère agitation pendant 16 h, on lave le support dans un tampon 0,1 M phosphate de sodium pH6. Le support présente finalement 20 mg de protéine de tannase par g de support sec.

### Exemple 2

On traite 40 ml de substrat constitué de 20 mM de gallate de méthyle dans un tampon 50mM acétate de sodium pH 5, avec 150 mg en équivalent de poids sec de tannase immobilisée de l'exemple 1 dans un réacteur traditionnel du type cuve agitée. La quantité de tannase est ainsi ajustée pour obtenir 75% d'hydrolyse du substrat en 30 min.

Ce réacteur comprend en particulier un corps tubulaire en verre dont la partie inférieure comprend un filtre de porosité d'environ 40µm connecté à une pompe péristaltique destinée à recueillir le produit hydrolysé, et dont la partie supérieure comprend la sortie d'une pompe péristaltique destinée à l'introduction du substrat. Le réacteur est immergé dans un bain d'eau à 30°C.

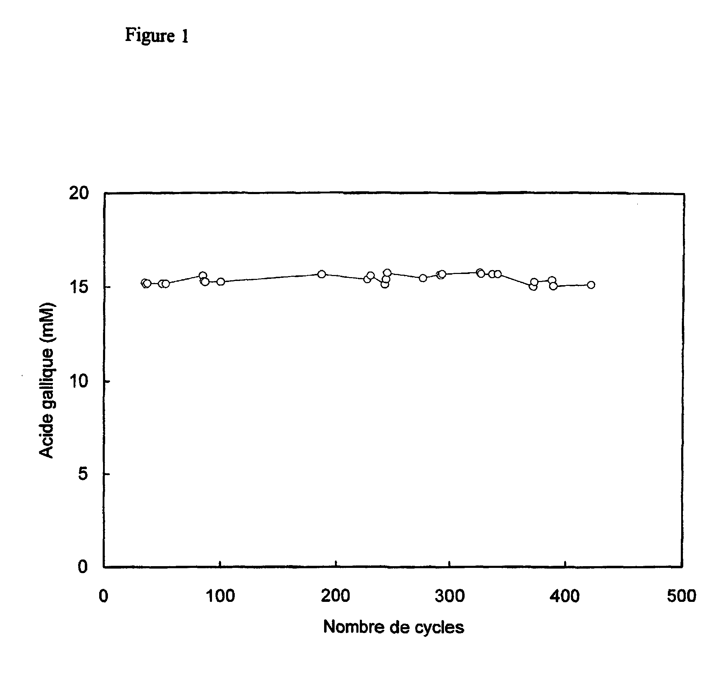

Dans un premier temps, on introduit le substrat dans le réacteur pendant environ 110 s, puis on le mélange pendant 27 min et 20 s, et enfin on recueille le milieu réactionnel pendant 50 s. On réalise 420 cycles successifs d'environ 30 min, et on mesure pour chaque cycle, par la méthode HPLC décrite ci-dessus, la quantité d'acide gallique libérée dans le milieu réactionnel. Les résultats présentés à la figure 1 montre que la tannase immobilisée de l'exemple 1 conserve 100% de son activité après 420 cycles d'hydrolyse successifs à 30°C.

### Exemple 3

On mélange 1g d'Eupergit-C® sec à 20 ml d'un tampon 0,3 M phosphate de sodium comprenant 0,175M d'éthylène diamine, pH8, on incube le mélange à température ambiante sous légère agitation pendant 16 h, on le lave abondamment avec de l'eau distillée, puis avec un tampon 0,1 M phosphate de sodium pH6. On obtient de l'Eupergit-C® modifié par l'éthylène diamine.

On mélange 1 g d'équivalent en poids sec d'Eupergit-C® humide modifié à 5,43 ml de la solution de tannase oxydée décrite à l'exemple 1, on incube le mélange à température ambiante sous une légère agitation et pendant 16 h, puis on lave le support dans un tampon 0,1 M phosphate de sodium pH6. On obtient de l'Eupergit-C® comprenant 25 mg de protéine de tannase par g de support sec.

### Exemple 4

On traite à 30°C 40 ml de substrat de l'exemple 2 avec 148mg en équivalent de poids sec de tannase immobilisée de l'exemple 3 dans le réacteur décrit à l'exemple 2. Cette quantité est réduite à 88mg pour les cycles de 35 à 65°C.

On réalise successivement 139 cycles à 30°C, 61 cycles à 35°C, 189 cycles à 40°C, 42 cycles à 45°C, 10 cycles à 50°C, 37 cycles à 55°C, 8 cycles à 60°C et 8 cycles à 65°C.

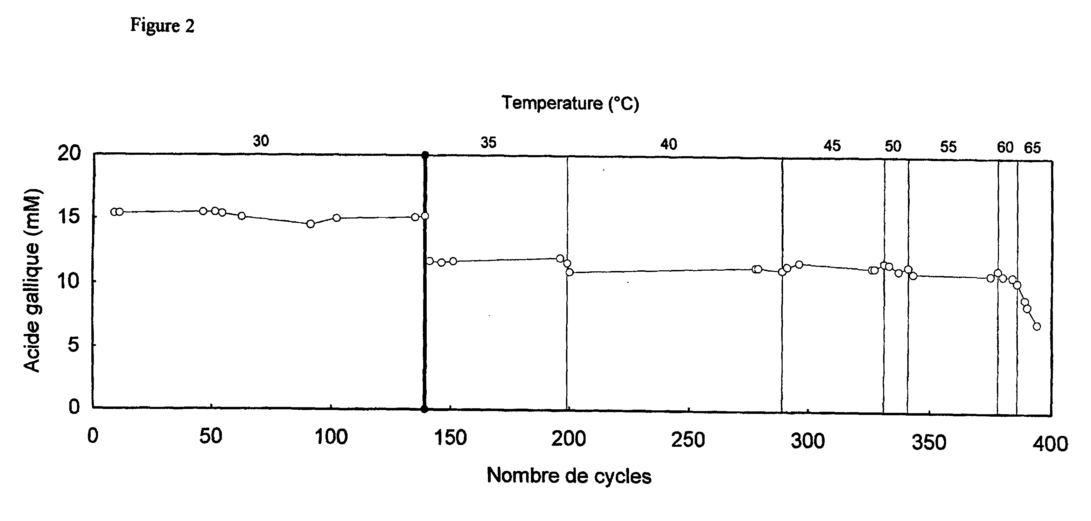

Les résultats présentés à la figure 2 sont similaires à ceux présentés à la figure 1, à savoir la tannase immobilisée de l'exemple 3 conserve 100% de son activité après 139 cycles d'hydrolyses successifs à 30°C. On peut néanmoins remarquer la surprenante stabilité dans le temps et à la température de la tannase immobilisée de l'exemple 3. En effet, l'enzyme conserve pratiquement 100% de son activité jusqu'à 60°C dans des conditions d'utilisation intensives de l'enzyme. Enfin à 65°C l'enzyme perd lentement son activité au cours du temps.

Pour comparaison, la tannase libre perd rapidement son activité à une température supérieure à 40°C (Thomas et al.).

### Exemple 5

On prépare un extrait de thé en mélangeant 1 kg de feuilles de thé noir de bonne qualité (Darjeeling FOP) à 15 litres d'eau déionisée à 95°C pendant 10 min et en séparant les feuilles de thé par filtration à chaud au travers d'un filtre de 40µm. On obtient ainsi un extrait contenant 2,29% de matières solides.

On prépare de la tannase immobilisée en plus grandes quantités selon le procédé décrit à l'exemple 3, et on la rince plusieurs fois avec l'extrait de thé ci-dessus afin de saturer et d'équilibrer la préparation.

On ajoute à 1 litre d'extrait à 55°C 15 g de cette tannase immobilisée humide. On agite le mélange pendant 1 h à 55°C puis on sépare l'extrait par filtration à travers un filtre de 40 µm. On récupère ainsi la tannase immobilisée pour la réutiliser 3 fois de suite avec le même extrait initial et dans les mêmes conditions. Les extraits sont ajustés à pH 5 avec une solution d'hydroxyde de sodium puis refroidis à 4°C et l'on mesure la quantité de matières insolubles formées que l'on compare avec celles produites dans l'extrait initial non traité.

Le tableau 2 ci-dessous indique les résultats obtenus. On constate que le traitement a permis une réduction importante de la quantité de crème de thé et cela même après réutilisation de la tannase immobilisée.

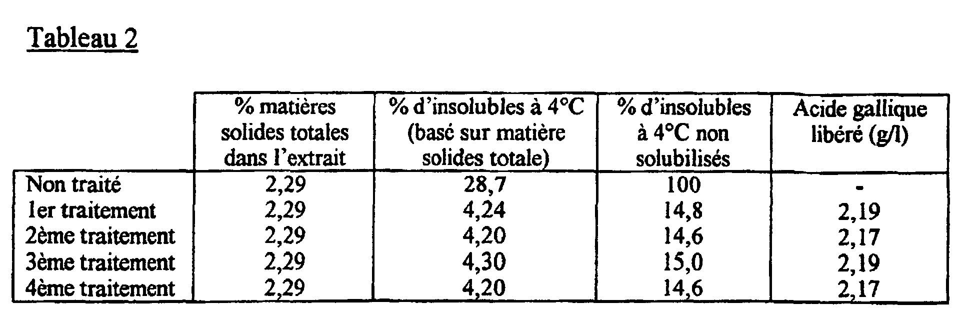

Les quatre extraits traités sont réunis et centrifugés à froid à 10'000 tours/min. pendant 15 min. Le surnageant est évaporé puis séché par lyophilisation. On obtient ainsi une poudre de thé qui présente par reconstitution une très bonne solubilité dans l'eau froide, la couleur brun- rouge typique d'un extrait de thé et le goût caractéristique d'un thé noir qui a conservé son astringence naturelle. La turbidité de la boisson est inférieure à 30 UTN.

### Exemple 6

On utilise l'extrait de feuilles de thé noir de l'exemple 5 que l'on passe dans une colonne maintenue à 55°C contenant 25 g de tannase immobilisée humide de l'exemple 5. L'extrait est pompé à la vitesse de 1 l/h et, après équilibre, est recueilli à la sortie de la colonne. Un aliquot de 1 est ajusté à pH 5 avec une solution d'hydroxyde de sodium puis refroidit à 4°C.

Après centrifugation à froid, le surnageant est séparé, concentré et lyophilisé. On obtient ainsi une poudre qui est complètement soluble à froid et qui présente le goût et la couleur désirés par les dégustateurs. La turbidité de la boisson est inférieure à 30 UTN.

## Revendications

1. Tannase immobilisée covalemment par sa partie glycosidique sur un support insoluble.

2. Tannase immobilisée selon la revendication 1, immobilisée sur un support à raison d'au moins 10 mg de tannase par g de support.

3. Tannase immobilisée selon la revendication 1, dont le support est saturé par un extrait de thé.

4. Tannase immobilisée selon la revendication 7, dont le support présente des pores de 20-2000nm.

5. Tannase immobilisée selon la revendication 1, dont le support est choisi dans le groupe formé par les supports organiques notamment du type protéine, polysaccharide, cellulose, dextran ou chitine, les supports de polymères synthétiques par exemple du type polystyrène, polyacrylate, polyméthacrylate, polyvinyle ou polyamide, et les supports inorganiques notamment du type silice, verre, oxyde métallique ou minéral naturel.

6. Procédé de préparation d'un extrait de thé, dans lequel on prépare un extrait aqueux de feuilles de thé, et on traite l'extrait à une température de 20-65°C par des tannases dont la partie glycosidique est immobilisée covalemment sur un support insoluble.

7. Procédé selon la revendication 6, dans lequel on traite l'extrait de thé par des tannases immobilisées selon l'une des revendications 2 à 5.

8. Procédé selon l'une des revendications 6 à 7, dans lequel on prépare un extrait de feuilles de thé, on refroidit l'extrait, on sépare la crème de thé, on la redissout par chauffage, on la traite par des tannases immobilisées selon l'une des revendications 1 à 5.

9. Procédé selon l'une des revendications 6 à 8, dans lequel on sèche l'extrait de thé traité par les tannases immobilisées.

10. Procédé selon l'une des revendications 6 à 9 dans lequel, on prépare un extrait aqueux de feuilles de thé, et on traite l'extrait à une température de 50-65°C dans une cuve comprenant en suspension des tannases dont la partie glycosidique est immobilisée covalemment sur un support insoluble.

11. Procédé selon l'une des revendications 6 à 9, on prépare un extrait aqueux de feuilles de thé, et on traite l'extrait à une température de 50-65°C en continu dans un réacteur comprenant un lit fixe de tannases immobilisées.

12. Procédé selon l'une des revendications 6 à 9, on prépare un extrait aqueux de feuilles de thé, et on traite l'extrait à une température de 50-65°C en continu dans un réacteur comprenant un lit fluidisé de tannases immobilisées

## Patentansprüche

1. Tannase, die über ihren Glykosidteil an einem unlöslichen Träger kovalent immobilisiert ist.

2. Immobilisierte Tannase nach Anspruch 1, die an einem Träger in einem Verhältnis von mindestens 10 mg Tannase pro g Träger immobilisiert ist.

3. Immobilisierte Tannase nach Anspruch 1, deren Träger mit einem Teeextrakt gesättigt ist.

4. Immobilisierte Tannase nach Anspruch 7, deren Träger Poren von 20-2000 nm aufweist.

5. Immobilisierte Tannase nach Anspruch 1, deren Träger aus der Gruppe ausgewählt ist, die von den organischen Träger insbesondere vom Typ Protein, Polysaccharid, Cellulose, Dextran oder Chitin, den Trägern aus synthetischen Polymeren beispielsweise vom Typ Polystyrol, Polyacrylat, Polymethacrylat, Polyvinyl oder Polyamid und den anorganischen Trägern insbesondere vom Typ Siliciumoxid, Glas, Metalloxid oder natürliches Mineral gebildet wird.

6. Verfahren zur Herstellung eines Teeextrakts, bei dem man einen wässrigen Extrakt von Teeblättern herstellt und den Extrakt bei einer Temperatur von 20-65 °C mit Tannasen behandelt, deren Glykosidteil an einem unlöslichen Träger kovalent immobilisiert ist.

7. Verfahren nach Anspruch 6, bei dem man den Teeextrakt mit immobilisierten Tannasen gemäß einem der Ansprüche 2 bis 5 behandelt.

8. Verfahren nach einem der Ansprüche 6 bis 7, bei dem man aus Teeblättern einen Extrakt herstellt, den Extrakt kühlt, den "Teerahm" abtrennt, ihn durch Erhitzen wieder auflöst und ihn mit immobilisierten Tannasen gemäß einem der Ansprüche 1 bis 5 behandelt.

9. Verfahren nach einem der Ansprüche 6 bis 8, bei dem man den mit den immobilisierten Tannasen behandelten Teeextrakt trocknet.

10. Verfahren nach einem der Ansprüche 6 bis 9, bei dem man einen wässrigen Extrakt von Teeblättern herstellt und den Extrakt bei einer Temperatur von 50 - 65 °C in einem Behälter behandelt, der Tannasen in Suspension enthält, deren Glykosidteil an einem unlöslichen Träger kovalent immobilisiert ist.

11. Verfahren nach einem der Ansprüche 6 bis 9, bei dem man einen wässrigen Extrakt von Teeblättern herstellt und den Extrakt bei einer Temperatur von 50 - 65 °C kontinuierlich in einem Reaktor behandelt, der ein Festbett von immobilisierten Tannasen enthält.

12. Verfahren nach einem der Ansprüche 6 bis 9, bei dem man einen wässrigen Extrakt von Teeblättern herstellt und den Extrakt bei einer Temperatur von 50 - 65 °C kontinuierlich in einem Reaktor behandelt, der ein Wirbelbett von immobilisierten Tannasen enthält.

## Claims

1. Tannase immobilised covalently by its glycoside portion on an insoluble substrate.

2. Immobilised tannase according to claim 1, immobilised on a substrate in an amount of at least 10 mg per g of substrate.

3. Immobilised tannase according to claim 1, of which the substrate is saturated with a tea extract.

4. Immobilised tannase according to claim 7, of which the substrate has 20-2000 nm pores.

5. Immobilised tannase according to claim 1, of which the substrate is chosen from the group formed of organic substrates, in particular of the protein, polysaccharide, cellulose, dextran or chitin type, synthetic polymer substrates for example of the polystyrene, polyacrylate, polymethacrylate, polyvinyl or polyamide type, and inorganic substrates in particular of the silica, glass, metal oxide or natural mineral type.

6. Method for preparing a tea extract, wherein an aqueous extract is prepared of tea leaves, and the extract is treated at a temperature of 20-65°C by tannases of which the glucoside portion is immobilised covalently on an insoluble substrate.

7. Method according to claim 6, wherein the tea extract is treated with immobilised tannases according to one of claims 2 to 5.

8. Method according to either of claims 6 or 7, wherein an extract of tea leaves is prepared, the extract is cooled, the tea cream is separated, redissolved by heating and treated by immobilised tannases according to one of claims 1 to 5.

9. Method according to one of claims 6 to 8, wherein the tea extract treated by the immobilised tannases is dried.

10. Method according to one of claims 6 to 9, wherein an aqueous extract of tea leaves is prepared and the extract is treated at a temperature of 50-65°C in a vat containing suspended tannases of which the glycoside portion has been immobilised covalently on an insoluble substrate.

11. Method according to one of claims 6 to 9, an aqueous extract of tea leaves being prepared and the extract being treated at a temperature of 50-65°C continuously in a reactor comprising a fixed bed of immobilised tannases.

12. Method according to one of claims 6 to 9, an aqueous extract of tea leaves being prepared and the extract being treated at a temperature of 50-65°C continuously in a reactor comprising a fluidised bed of immobilised tannases.
